# EUROPÄISCHE PATENTSCHRIFT

(11) **EP 1 347 764 B1**
(45) Veröffentlichungstag und Bekanntmachung des Hinweises auf die Patenterteilung: **14.03.2007**
(21) Anmeldenummer: 01985426.4
(22) Anmeldetag: 20.12.2001
(51) Int. Cl.: A61K 31/568, A61K 47/10, A61P 15/08

(54) **GELZUSAMMENSETZUNG AUF ALKOHOLISCHER BASIS ZUR BEHANDLUNG VON HYPOGONADISMUS DURCH TRANSSKROTALE APPLIKATION**
GEL COMPOSITION BASED ON ALCOHOL FOR THE TREATMENT OF HYPOGONADISM THROUGH TRANSSCROTAL APPLICATION
GEL A BASE D'ALCOOL POUR TRAITER L'HYPOGONADISME PAR VOIE TRANS-SCROTALE

(30) Priorität: 22.12.2000 DE 10064205
(43) Veröffentlichungstag der Anmeldung: 01.10.2003
(73) Patentinhaber: Dr. August Wolff GmbH & Co. KG Arzneimittel, 33611 Bielefeld (DE)
(72) Erfinder: SCHULZE, Bernd,Dr. August Wolff Gmbh & Co., 33611 Bielefeld (DE); NIESCHLAG, Eberhard, 48167 Münster (DE)
(74) Vertreter: Grünecker, Kinkeldey, Stockmair & Schwanhäusser Anwaltssozietät
(86) Internationale Anmeldenummer: PCT/EP2001/015123
(87) Internationale Veröffentlichungsnummer: WO 2002/051421

(56) Entgegenhaltungen:
- EP-A- 0 043 738
- WO-A-02/11768
- WO-A-97/41865
- WO-A-97/43989
- WO-A-99/24041
- DE-C- 19 803 193
- US-A- 4 867 982
- US-A- 5 776 923
- US-A- 5 968 919
- FINDLAY J C ET AL: "TREATMENT OF PRIMARY HYPOGONADISM IN MEN BY THE TRANSDERMAL ADMINISTRATION OF TESTOSTERONE" JOURNAL OF CLINICAL ENDOCRINOLOGY & METABOLISM, Bd. 68, Nr. 2, 1989, Seiten 369-373, XP001088685 ISSN: 0021-972X
- LIN S S ET AL: "Transdermal testosterone delivery: Comparison between scrotal and nonscrotal delivery systems" PHARMACEUTICAL DEVELOPMENT AND TECHNOLOGY, (AUG 1999) VOL. 4, NO. 3, PP. 405-414. PUBLISHER: MARCEL DEKKER INC, 270 MADISON AVE, NEW YORK, NY 10016. ISSN: 1083-7450., XP001084425 RUTGERS STATE UNIV, COLL PHARM, CONTROLLED DRUG DELIVERY RES CTR, POB 10406, NEW BRUNSWICK, NJ 08906 (Reprint);RUTGERS STATE UNIV, COLL PHARM, CONTROLLED DRUG DELIVERY RES CTR, NEW BRUNSWICK, NJ 08906
- "Dictonnaire Vidal" 1982 XP002205868 Seite 967, Spalte 1
- KIM E D ET AL: "Papaverine topical gel for treatment of erectile dysfunction." THE JOURNAL OF UROLOGY. UNITED STATES FEB 1995, Bd. 153, Nr. 2, Februar 1995 (1995-02), Seiten 361-365, XP001084767 ISSN: 0022-5347
- ODAME I ET AL: "Early diagnosis and management of 5 alpha-reductase deficiency." ARCHIVES OF DISEASE IN CHILDHOOD. ENGLAND JUN 1992, Bd. 67, Nr. 6, Juni 1992 (1992-06), Seiten 720-723, XP001088867 ISSN: 1468-2044

## Beschreibung

Die vorliegende Erfindung betrifft die Verwendung einer Gelzusammensetzung zur Behandlung und/oder Prophylaxe von Hypogonadismus.

Zur Ausbildung der männlichen Geschlechtsmerkmale sind die männlichen Sexualhormone, die Androgene, verantwortlich. Außerdem sind sie für die Fortpflanzung unabdingbar. Hauptvertreter der Androgene ist das Testosteron, welches unerlässlich für die Entwicklung und Funktion der inneren und äußeren männlichen Geschlechtsorgane ist, einen fördernden Einfluss auf das Muskelwachstum ausübt, die Verteilung und Dichte der Körperbehaarung steuert, einen positiven Einfluss auf die Ausschüttung von Erythropoetin bei der Erythrozytenproduktion ausübt und kognitive Funktionen beeinflusst. Bei Testosteronmangel (Hypogonadismus) unterscheidet man im wesentlichen zwei Formen, die als primärer und als sekundärer Hypogonadismus bezeichnet werden. Eine fehlende oder mangelhafte körpereigene Testosteronproduktion, die ihren Ursprung in einer Fehlfunktion der Hoden, dem Hauptsyntheseort von Testosteron, hat, wird als primärer Hypogonadismus bezeichnet. Liegt die Ursache in einer Erkrankung des Hypothalamus oder der Hypophyse, spricht man von einem sekundären Hypogonadismus. Eine Indikation für die Therapie beider Formen des Hypogonadismus ist angezeigt, wenn bei Symptomen des Androgenenmangels, wie zum Beispiel bei Erkrankungen infolge von Testosteronmangel, wie Osteoporose, Muskelatrophie, Ausfallerscheinung in der Seneszenz, Verlust von Libido und Potenz, Depressionen und Anämien, die in den Morgenstunden gemessene Testosteronkonzentration im Serum unter 12 nmol/l abfällt. Bei der Behandlung handelt es sich um eine Substitutionstherapie, deren Effektivität direkt an den Testosteronkonzentrationen im Serum abgelesen werden kann. Unmittelbares Ziel einer Testosteronsubstitution besteht darin, Testosteronkonzentrationen im Serum im Normalbereich zu erzielen.

Chemisch leitet sich Testosteron, 17-β-Hydroxy-androst-4-en-3-on, von der Grundstruktur aller Androgene, dem Androstan, ab. Seine spezifische biologische Aktivität verdankt Testosteron der Ketogruppe in Position 3, der Doppelbindung in Position 4 und der Hydroxygruppe in Position 17 des Androstan-Grundgerüstes. Um Testosteron in der Therapie einsetzen zu können, kann es als solches oder chemisch modifiziert, wie zum Beispiel durch Veresterung in Position 17, eingesetzt werden. Insbesondere ist für eine Therapie mit einem Testosteronpräparat die Applikationsform von Bedeutung. Testosteronimplantate, orale Testosteronpräparate, intramuskuläre Testosteronpräparate und transdermale Testosteronpräparate finden Verwendung.

Eine Übersicht zum Thema "Therapie mit Testosteron" veröffentlichten E. Nieschlag und H.M. Behre in Andrologie, Grundlagen und Klinik der reproduktiven Gesundheit des Mannes, S. 315-329, Springer Verlag 1996.

Testosteronimplantate bestehen aus reinem Testosteron, das in zylindrische Formen von 12 mm Länge und 4,5 mm Durchmesser gegossen ist. Ein Implantat enthält 200 mg. Wenn 3 bis 6 Implantate appliziert werden, werden langsam abfallende Serumtestosteronspiegel im Normalbereich für 4 bis 6 Monate erreicht. Der zur Implantation notwendige, operative Eingriff, die bei 5% der Patienten zu beobachtenden Extrusionen der Implantate und gelegentliche Blutungen und Infektionen bilden gravierende Nachteile der Testosteronimplantate. Entsprechende Präparate sind nur in Großbritannien und Australien auf dem Markt.

Oral verabreichtes, freies Testosteron wird in der Leber vollständig metabolisiert, so dass es die Zielorgane nicht erreicht. In der oralen Therapie kommen deshalb Präparate mit chemisch modifiziertem Testosteron, wie in Position 17β mit Undekansäure verestertes Testosteron oder in 17α Position methyliertes Testosteron zum Einsatz.

Wird natürliches Testosteron intramuskulär verabreicht, hat es eine nur sehr kurze Halbwertszeit. Um die Wirkungsdauer zu verlängern, wurde Testosteron in Position 17 mit aliphatischen Seitenketten verestert. Die am weitesten verbreitete Substitutionstherapie besteht in der intramuskulären Verabreichung von Testosteron-Enanthat, das eine terminale Halbwertzeit von 4,5 Tagen aufweist. Die Standarddosierung beträgt 250 mg Testosteron-Enanthat. Es werden sehr schnell hohe Testosteronserumkonzentrationen von bis zu 50 nmol/l erreicht, welche allmählich abfallen, um etwa am 12. Tag die untere Normalgrenze zu passieren. Bei wiederholten Injektionen, wie sie für die Substitutionstherapie erforderlich sind, entsteht somit ein Sägezahnprofil, bei dem je nach Injektionsintervall Phasen mit supraphysiologischen, physiologischen und infraphysiologischen Werten aufeinander folgen. Während diese Substitutionsform für die Aufrechterhaltung der biologischen Wirkung des Testosterons ausreichend ist, empfindet der Patient die starken Schwankungen als störend, da das allgemeine Wohlbefinden, die Stimmung und die sexuelle Aktivität diesem Auf und Ab folgen.

Mit der Einführung transdermaler Testosteronpräparate ist die Therapie von Testosteronmangelerkrankungen um weitere, interessante Alternativen bereichert worden. Im endokrinologischen Bereich hat insbesondere die transdermale Applikation von Östrogenen zur Behandlung klimakterischer Ausfallerscheinungen bei der Frau weite Verbreitung gefunden. Die Entwicklung eines transdermalen Systems zur Substitutionstherapie des hypogonadalen Mannes stieß jedoch auf Schwierigkeiten, da beim Mann Dosen im Bereich der normalen Eigenproduktion von Testosteron, das heißt etwa 6 mg pro Tag, appliziert werden müssen, während die entsprechende Dosis für Östradiol bei einer Frau im Mikrogrammbereich liegt.

Unterschiedliche Hautpartien weisen unterschiedliche Resorptionsfähigkeit auf. Da wegen der physiologischen Aufgabe des Temperaturausgleichs die Skrotalhaut besonders stark und bis in die obersten Epithelschichten durchblutet ist, hat diese Hautpartie ein besonders hohes Resorptionsvermögen (im Vergleich zur Haut des Unterarms etwa 40 mal höher). Diese Besonderheit wurde bei der Entwicklung eines transskrotalen Applikationssystems ausgenutzt. Zum Einsatz kommen Testosteronpflaster (Testoderm®) aus 40 oder 60 cm² großen, aus Polymer bestehenden Membranen, die mit 10 bzw. 15 mg reinem natürlichen Testosteron beladen sind. Wenn diese Membranen auf das Skrotum appliziert werden, geben sie genügend Testosteron in den Kreislauf ab, um physiologische Serumtestosteronspiegel für einen Tag zu garantieren. Wenn die Membranen in den Morgenstunden angebracht werden, kann sogar der physiologische Tagesrhythmus des Testosterons imitiert werden. Die Applikation eines Testosteronpflasters erfordert jedoch eine entsprechende Vorbereitung. Um einen engen Hautkontakt zu garantieren, muss das Skrotum von Zeit zu Zeit durch Rasur oder mit der Schere von Haaren befreit werden. Da das Pflaster keine Klebmittel enthält, muss es zur hinreichenden Fixierung auf dem Skrotum vor seiner Anwendung mit einem Haarföhn aufgewärmt werden. Bei Ablösung des Pflasters muss es erneut aufgewärmt und auf das Skrotum aufgebracht werden. Das Testosteronpflaster auf dem Skrotum wird von vielen Patienten als unbequem empfunden. Jucken und eine Reizung der Skrotalhaut sind weitere, gravierende Nachteile dieser Testosterontherapie.

Es wurden auch transdermale Testosteronpflaster entwickelt, die auf die nicht-skrotale Haut (z.B. Bauch, Oberarm, Oberschenkel, Brust oder Rücken) appliziert werden können (Androderm®). Um die erforderliche Testosteronmenge durch die Haut transportieren zu können, sind diese Systeme mit einem Resorptionsbeschleuniger (Enhancer) ausgestattet. Dies führt bei einem hohen Prozentsatz zu dermalen Irritationen, die sich unter anderem durch schwere bulllöse Hautveränderungen äußern können. Die Testosteronpflaster sind optisch auffällig und lassen keine flexible Dosierung zu.

Ferner sind transdermale Anwendungen von Testosteron in Gelform bekannt. In Frankreich ist das transdermale Präparat Andractim®, das 5α-Dihydrotestosteron in einer Konzentration von 2,5% in einem hydroalkoholischen Gel enthält, bekannt. Wenn dieses Gel auf genügend große Hautflächen (Arme, Schultern, Brust, Bauch oder Oberschenkel) aufgetragen wird, penetriert das Dihydrotestosteron in die Haut und im Serum können supraphysiologische Dihydrotestosteron-Werte gemessen werden.

Ein weiteres dermales Therapiesystem auf Basis eines testosteronhaltigen hydroalkoholischen Gels, welches 1 Gew.-% Testosteron enthält, ist vor kurzem von der amerikanischen Zulassungsbehörde FDA zugelassen worden, und wird von der Firma Unimed Pharmaceuticals, lnc. unter dem Handelnamen AndroGel® vertrieben. Auch dieses Produkt wird im Bereich der Oberarme, Schultem und/oder Vorderseite des Rumpfes aufgetragen. Allerdings erscheint die Applikation eines Gels auf eine große Hautfläche unpraktikabel, da über Haut- und Wäschekontakt unbeabsichtigte Testosteronaufnahme bei der Partnerin erfolgen kann, was zu unerwünschten Virilisierungserscheinungen führen kann. Diese Therapie macht ein Abwaschen des aufgetragenen Gels vor sexuellen und sozialen Kontakten mit andersgeschlechtlichen Personen erforderlich.

Die US 5,776,923 beschreibt Arzneimittel, enthaltend 7 bis 20% DHEA zur Behandlung von Hypogonadismus, beispielsweise in der Form einer Lotion oder in der Form eines Gels. Die DE 198 03 193 A beschreibt ein Gel, enthaltend 2,5% Testosteron. Die Behandlung von Hypogonadismus und die Anwendung auf dem Skrotum sind nicht offenbart. Die WO 99/24041 beschreibt Gele mit einem Testosterongehalt von 2%. Diese Entgegenhaltung betrifft nicht die Behandlung von Hypogonadismus und offenbart keine transskrotale Anwendung. Die US 5,968,919 beschreibt topisch anzuwendende Zusammensetzungen, die Testosteron enthalten können. J.C. Findlay et al. beschreiben in Journal of Clinical Endocrinology and Metabolism, Vol. 68, Nr. 2, 369 ff. die Behandlung von Hypogonadismus mit einer testosteronimprägnierten Membran.

S. Lin et al. beschreiben in Pharmaceutical Development and Technology, 4 (3), 405-414 (1999) die Behandlung von Hypogonadismus mit transdermalen Verabreichungssystemen. Dictionnaire Vidal, 1982, beschreibt eine testosteronenthaltene Lösung zur Behandlung von Hypogonadismus. Diese Lösung wird auf den Thorax/Abdomen aufgetragen.

Die WO 97/41865 beschreibt Arzneimittel zur topischen skrotalen Applikation zur Behandlung der erektilen Dysfunktion. Die US 4,867,982 beschreibt eine Vorrichtung, die Testosteron enthält, zur Anwendung auf dem Skrotum zur Behandlung von Hypogonadismus. Dadurch soll beispielsweise eine Applikation von Gelen ersetzt werden. Die WO 97/43989 beschreibt Zusammensetzungen und Vorrichtungen zur transdermalen Hormontherapie.

Die WO 02/11768 beschreibt eine pharmazeutische Zusammensetzung, geeignet zur transdermalen Verabreichung von aktiven Bestandteilen. Die WO 03/047548 beschreibt eine Gelzusammensetzung oder Lösung, umfassend Dihydrotestosteron und mindestens einen Permeationsverstärker.

Aufgabe der vorliegender Erfindung ist es, die vorstehend genannten Probleme zu lösen und eine Therapie möglichkeit zur Verfügung zu stellen, die zur Behandlung und/oder Prophylaxe von Hypogonadismus, sowie den damit verbundenen vorstehend genannten Symptomen, geeignet ist.

Diese Aufgabe wird erfindungsgemäß gelöst durch die Verwendung einer Gelzusammensetzung auf alkoholischer Basis, enthaltend mindestens ein androgenes Steroid sowie mindestens ein C₃ bis C₄-Diol als Resorptionsverstärker, wie in Anspruch 1 definiert. Bevorzugte Ausführungsformen sind in Ansprüche 2 bis 8 aufgeben.

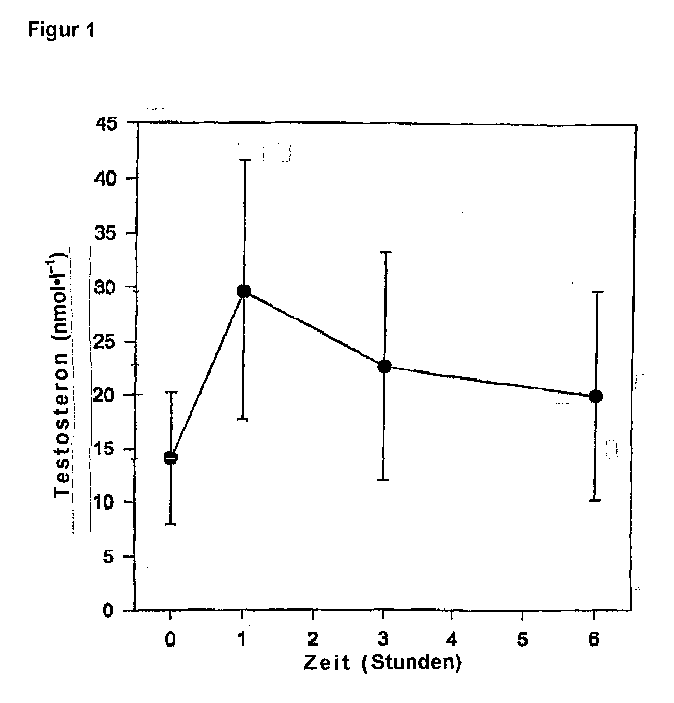

Fig. 1 zeigt die gemessene mittlere Konzentration von Testosteron im Serum 0-6 Stunden nach skrotaler Applikation von 1 g eines Testosterongels.

Überraschenderweise wurde gefunden, dass die erfindungsgemäße Verwendung einer Gelzusammensetzung eine besonders gute Penetration des androgenen Steroids durch die Haut in das Blut gewährleistet. Insbesondere hat sich herausgestellt, dass im Gegensatz zu allen bisher bekannten Testosteronzusammensetzungen, wie Testosterongels, die erfindungsgemäß verwendete Gelzusammensetzung ca. 10 Minuten nach dem Auftragen abgewaschen werden kann, ohne dass der Penetrationsprozess damit eingeschränkt wird. Im Gegenteil, es kommt offenbar zu einer Intensivierung dieses Penetrationsprozesses. Es wurde somit gefunden, dass der mechanische Faktor des Abwaschens offenbar eine zusätzliche penetrationsbeschleunigende Wirkung verursacht. Es konnte in diesem Zusammenhang nachgewiesen werden, dass nach dem Auftragen der Getzusamrnensetzung und anschließendem Abwaschen nach ca. 10 Minuten trotzdem ein deutlicher Anstieg des Testosterorispiegels im Blut nachweisbar war, der zur Normalisierung der hypogonadalen Werte führte. Durch die Möglichkeit, dass die Gelzusammensetzung ca. 10 Minuten nach Auftragen abgewaschen werden kann, kann noch besser gewährleistet werden, dass es zu keiner Kontamination andersgeschlechtlicher oder kindlicher Personen bei sexuellem bzw. sozialem Kontakt kommt. Gleichzeitig wird der Therapieerfolg bei der Person, die die Gelzusammensetzung verwendet hat, nicht gefährdet.

Die Gelzusammensetzung gewährleistet somit eine verbesserte Resorption des Wirkstoffes in kurzer Zeit. Dieses Phänomen wird durch ein Abwaschen der Gelzusammensetzung nach relativ kurzer Zeit nicht abgeschwächt, sondern vielmehr verstärkt. Dabei tritt innerhalb kurzer Zeit ausreichend Wirkstoff in die Blutbahn ein, so dass es zu einem effektiven Hormonanstieg kommt, während gleichzeitig eine Übertragung des Wirkstoffes bei engem körperlichen Kontakt mit andersgeschlechtlichen Personen bzw. Kindern und den damit zu befürchtenden den Folgen einer Vermännlichung verhindert werden kann.

Erfindungsgemäß eingesetzte, mit androgenem Steroid bezeichnete Verbindungen umfassen natürliche und synthetische androgene Steroide, welche eine Verminderung der Symptome von Testosteronmangel bei Männern bewirken. Bevorzugt werden die erfindungsgemäß eingesetzten androgenen Steroide ausgewählt aus der Gruppe, bestehend aus Testosteron, Testosteronestem, Methyltestosteron, Methyltestosteronestern, Androstendion, Andrenosteron, Dehydroepiandrosteron, Fluoxymesteron, Methandrostenolon, 17α-Methylnortestosteron, Norethandrolon Dihydrotestosteron, Oxymetholon, Stanozolol, Ethylestrenol, Oxandrolon, Bolasteron und Mesterolon. Von dieser Gruppe sind Testosteron, Testosteronester, Methyltestosteron, Dihydrotestosteron oder Gemische davon bevorzugt. Testosteronester können Propionate, Phenylacetate, Enanthate, Undekanoate, Cypionate oder Buciclate sein. Besonders bevorzugt ist Testosteron.

Der Steroidgehalt der Gelzusammensetzung beträgt 0,1 bis 4 Gew.%, und am meisten bevorzugt 2 bis 3 Gew.%, jeweils bezogen auf das Gewicht der Gelzusammensetzung.

Bei dem C₃ bis C₄-Diol handelt es sich üblicherweise um Propylenglykol, Butylenglykole, einschließlich 1,3-Dihydroxybutan, 2,3-Dihydroxybutan und 1,4-Dihydroxybutan sowie Mischungen dieser Diole. Vorzugsweise wird Propylenglykol allein oder zusammen mit mindestens einem Butylenglykol eingesetzt.

Die Gelzusammensetzung enthält üblicherweise das mindestens eine C₃ bis C₄-Diol in einer Menge von 0,1 bis 20 Gew.%, besonders bevorzugt 0,5 bis 10 Gew.%, noch bevorzugter 1 bis 10 Gew.-% und am meisten bevorzugt 3 bis 8 Gew.%, jeweils bezogen auf das Gewicht der Gelzusammensetzung.

Die Gelzusammensetzung wird auf alkoholischer Basis formuliert. Beispiele des Alkohols beinhalten C₂ bis C₇-Alkohole, vorzugsweise C₂ bis C₄-Alkohole, insbesondere bevorzugt Ethanol und Propanol.

Die Gelzusammensetzung wird so formuliert, dass die Gelzusammensetzung den Alkohol in einer Menge von 30 bis 90 Gew.%, bevorzugt 40 bis 70 Gew.%, am meisten bevorzugt 50 bis 60 Gew.%, jeweils bezogen auf das Gewicht der Gelzusammensetzung enthält.

Die Gelzusammensetzung enthält außerdem einen pH-Stabilisator, um eine homogene Zusammensetzung des Gels zu gewährleisten. Dabei können übliche pH-Stabilisatoren eingesetzt werden. Üblich sind schwache Säuren, schwache Basen, schwach saure Puffer und/oder schwach basische Puffer. Besonders geeignet sind schwach basische oder schwach saure Puffer. Insbesondere eignet sich als Puffer Tris-Puffer (α,α,α-Tris-(Hydroxymethyl)-methylamin, Tromethamol) oder Salze davon.

Der pH-Stabilisator ist in einer Menge vorhanden, um die Stabilität der Gelzusammensetzung zu gewährleisten, d.h. in einer Menge von 0,01 bis 1 Gew.-%, bevorzugt 0,05 bis 0,5 Gew.%, bezogen auf das Gewicht der Gelzusammensetzung.

Als weiteren Bestandteil enthält die Gelzusammensetzung ein Polymer auf Poly(meth)acrylsäurebasis, um vorzugsweise die Viskosität oder den Strukturaufbau der Gelzusammensetzung weiter zu verbessern. Bevorzugte Polymere werden aus der Gruppe der Carbomere ausgewählt, die ein Acrylsäuregrundgerüst und geringe Mengen Polyalkenylpolyether als Vemetzungsmittel enthalten, wobei Co-Monomere, wie C₁₀ bis C₃₀-Alkylacrylate ebenfalls enthalten sein können. Übliche Carbomere sind allgemein bekannt und beinhalten beispielsweise Carbopol 980, Carbopol 941, Carbopol 940, Carbopol 934, Carbopol Ultrez U 10 sowie Carbopol ETD-2020, die einzeln oder als Mischung eingesetzt werden können.

Der Gehalt dieses Polymers beträgt 0,1 bis 6 Gew.-%, bevorzugt 0,5 bis 4 Gew.-%, jeweils bezogen auf das Gewicht der Zusammensetzung.

Die Herstellung der Getzusammensetzung erfolgt üblicherweise, indem mindestens ein androgenes Steroid sowie mindestens ein C₃ bis C₄-Diol gegebenenfalls mit Hilfs-und/oder Trägerstoffen in eine geeignete Formulierungsform gebracht wird. Die Hilfs-und Trägerstoffe stammen aus der Gruppe der Trägermittel, Konservierungsstoffe und anderer üblicher Hilfsstoffe.

Die Gelzusammensetzungen werden auf dem Skrotum, zur Behandlung und/oder Prophylaxe von Hypogonadismus aufgetragen. Die mit Hypogonadismus verbundenen Testosteronmangelerkrankungen umfassen zum Beispiel Osteoporose, Muskelatrophie, Ausfallerscheinung in der Seneszenz, Verlust von Libido und Potenz, Depressionen und Anämien.

Der Gelzusammensetzung können beliebige übliche Trägerstoffe, Hilfsstoffe und gegebenenfalls weitere Wirkstoffe zugesetzt werden. Bevorzugte Hilfsstoffe stammen aus der Gruppe der Konservierungsstoffe, Antioxidantien, Stabilisatoren, Lösungsvermittler, Vitamine, Färbemittel und Geruchsverbesserer.

Die Gelzusammensetzung kann neben einem oder mehrerer androgener Steroide die üblichen Trägerstoffe enthalten, zum Beispiel tierische und pflanzliche Fette, Wachse, Paraffine, Stärke, Traganth, Cellulosederivate, Polyethylenglykole, Ethanol, Propanol, Polyacrylsäure, Silikone, Bentonite, Kieselsäure, Talkum und Zinkoxid oder Gemische dieser Stoffe.

Weitere Additive, wie Eindickungsmittel, Gummen und Mittel, welche die Löslichkeit androgener Steroide erhöhen, können in der Gelzusammensetzung enthalten sein. Besonders bevorzugt sind Verbindungen, welche die Löslichkeit androgener Steroide in hydrophilen Medien erhöhen, insbesondere Cyclodextrine, wie α-, β- und/oder γ-Cyclodextrin.

Die Menge, in der die Gelzusammensetzung auf der entsprechenden Körperstelle aufgetragen wird, beträgt bevorzugt 0,2 bis 20,0 g, besonders bevorzugt 1,0 bis 10,0 g und am meisten bevorzugt 1,0 bis 7,0 g.

Die Menge an Steroid in einer zu verabreichenden Dosis beträgt folglich üblicherweise 40 bis 400 mg, vorzugsweise 50 bis 300 mg, noch bevorzugter 70 bis 250 mg und am meisten bevorzugt 100 bis 250 mg.

Die Applikation der Gelzusammensetzung erfolgt bevorzugt mittels einer Tube, einer Weichgelatinekapsel oder mit einem Dosierspender mit oder ohne Aerosol.

Überraschenderweise wurde gefunden, dass bei der erfindungsgemäß auf dem Skrotum verwendeten Zusammensetzung bereits das Auftragen vergleichsweise geringer Mengen genügt, um einen dauerhaften, die zirkadianen Schwankungen des Serumtestosteronspiegels imitierenden Testosteronwert im Blut zu erreichen. Dieser Effekt ist durch das gute Resorptionsvermögen der Skrotalhaut nicht ausreichend zu erklären. Er beruht vielmehr im Gegensatz zu herkömmlichen transdermalen Therapiesystemen auf der Hodenmassage, die zwangsläufig beim Auftragen der Zusammensetzung auf das Skrotum erfolgt. Überraschenderweise wirkt sich dieser Zusatzeffekt im Sinne einer Synergie auf die exogene Testosteron-Applikation aus.

Die erforderliche Konzentration an androgenen Steroiden in der verwendeten Zusammensetzung, um einen normalen Testosteronspiegel im Serum zu erreichen, ist im Vergleich zu herkömmlichen transdermalen Applikationen sehr gering.

Angesichts der Probleme mit der transskrotalen Applikation von Testosteron-Pflastern sollte das Auftragen einer Zusammensetzung auf die empfindliche Skrotalhaut zu massiven Hautirritationen führen. Überraschenderweise wurde gefunden, dass diese nachteiligen Effekte bei der erfindungsgemäßen Verwendung einer Zusammensetzung auf dem Skrotum ausbleiben.

Bei der erfindungsgemäßen Verwendung der Zusammensetzung auf dem Skrotum bleibt im Gegensatz zur transdermalen Applikation, die im Bereich der Oberarme, der Oberschenkel, des Brust- bzw. des Bauchbereiches erfolgt, eine ungewollte Übertragung androgener Steroide auf die Partnerin bei intensivem Körperkontakt, wie z.B. beim Geschlechtsverkehr, weitgehend aus, und bei gewöhnlichen sozialen Kontakten mit Kindern oder Frauen wird eine Kontaminierung vollständig vermieden.

Erfindungsgemäß verwendete, mit androgenem Steroid bezeichnete Verbindungen umfassen natürliche und synthetische androgene Steroide, welche eine Verminderung der Symptome von Testosteronmangel bei Männern bewirken. Bevorzugt werden die erfindungsgemäß verwendeten androgenen Steroide ausgewählt aus der Gruppe, bestehend aus Testosteron, Testosteronestem, Methyltestosteron, Methyltestosteronestern, Androstendion, Andrenosteron, Dehydroepiandrosteron, Fluoxymesteron, Methandrostenolon, 17α-Methylnortestosteron, Norethandrolon Dihydrotestosteron, Oxymetholon, Stanozolol, Ethylestrenol, Oxandrolon, Bolasteron und Mesterolon. Von dieser Gruppe sind Testosteron, Testosteronester, Methyltestosteron, Dihydrotestosteron oder Gemische davon bevorzugt.

Testosteronester können Propionate, Phenylacetate, Enanthate, Undekanoate, Cypionate oder Buciclate sein. Besonders bevorzugt ist Testosteron.

Die Herstellung der erfindungsgemäß verwendeten Zusammensetzung erfolgt üblicherweise, indem mindestens ein androgenes Steroid gegebenenfalls mit Hilfs-und/oder Trägerstoffen in eine geeignete Fomlulierungsform gebracht wird. Die Hilfs-und Trägerstoffe stammen aus der Gruppe der Trägermittel, Konservierungsstoffe und anderer üblicher Hilfsstoffe.

Die Zusammensetzungen werden erfindungsgemäß auf dem Skrotum zur Behandlung und/oder Prophylaxe von Hypogonadismus angewendet. Die mit Hypogonadismus verbundenen Testosteronmangelerkrankungen umfassen zum Beispiel Osteoporose, Muskelatrophie, Ausfallerscheinung in der Seneszenz, Verlust von Libido und Potenz, Depressionen und Anämien.

Der Zusammensetzung können beliebige übliche Trägerstoffe, Hilfsstoffe und gegebenenfalls weitere Wirkstoffe zugesetzt werden. Bevorzugte Hilfsstoffe stammen aus der Gruppe der Konservierungsstoffe, Antioxidantien, Stabilisatoren, Lösungsvermittler, Vitamine, Färbemittel und Geruchsverbesserer.

Gele können neben einem oder mehrerer androgener Steroide die üblichen Trägerstoffe enthalten, zum Beispiel tierische und pflanzliche Fette, Wachse, Paraffine, Stärke, Traganth, Cellulosederivate, Polyethylenglykole, Ethanol, Propanol, Polyacrylsäure, Silikone, Bentonite, Kieselsäure, Talkum und Zinkoxid oder Gemische dieser Stoffe.

Weitere Additive, wie Eindickungsmittel, Gummen und Mittel, welche die Löslichkeit androgener Steroide erhöhen, können vorzugsweise in der erfindungsgemäß verwendeten Zusammensetzung enthalten sein. Besonders bevorzugt sind Verbindungen, welche die Löslichkeit androgener Steroide in hydrophilen Medien erhöhen, insbesondere Cyclodextrine, wie α-, β- und/oder γ-Cyclodextrin.

Der Steroidgehalt der erfindungsgemäß verwendeten Zusammensetzung beträgt 0,1 bis 4 Gew.-%, und am meisten bevorzugt 2 bis 3 Gew.%, jeweils bezogen auf das Gewicht der Zusammensetzung.

Die Menge der erfindungsgemäß auf dem Skrotum verwendeten Zusammensetzung beträgt bevorzugt 0,2 bis 20,0 g, besonders bevorzugt 1,0 bis 10,0 g und am meisten bevorzugt 1,0 bis 7,0 g.

Die Applikation der auf dem Skrotum erfindungsgemäß verwendeten Zusammensetzung erfolgt bevorzugt mittels einer Tube, einer Weichgelatinekapsel oder mit einem Dosierspender mit oder ohne Aerosol.

Die folgenden Beispiele erläutern die vorliegende Erfindung. Alle Verbindungen oder Komponenten, die in den Formulierungen verwendet werden können, sind entweder bekannt und käuflich erhältlich oder können nach bekannten Methoden synthetisiert werden.

### Beispiel 1

In Tabelle 1 sind Formulierungen für Gele (G-I, G-II und G-III), in Gewichtsteilen angegeben, welche erfindungsgemäß verwendet werden können.

**Tabelle 1**

| | **G-I** | **G-II** | **G-III** |
|---|---|---|---|
| **Testosteron** | 2,5 | 2,5 | 1,0 |
| **α-Bisabolol** | 1,0 | - - | |
| **Lecithin** | 1,5 | - - | |
| **Ethanol (96%)** | 51,5 | 56,0 | 56,0 |
| **Propylenglycol** | 5,0 | 5,0 | 5,0 |
| **Carbopol 980** | 1,0 | 1,0 | 1,0 |
| **Tromethamol** | 0,14 | 0,14 | 0,14 |
| **Wasser** | 37,36 | 35,36 | 36,86 |

Zur Evaluierung der perkutanen Testosteronresorption bei skrotaler Applikation wurde folgende Untersuchung durchgeführt:

Bei sechs freiwilligen Versuchspersonen wurde eine einheitliche Menge von 1 g Gel (G-II) um 8.00 Uhr morgens unter Verwendung von Einmalhandschuhen auf das Skrotum aufgetragen. Die Probanden wurden angewiesen, das Skrotum nicht vor Ablauf von 6 Stunden nach dem Auftragen zu waschen und Hautkontakte der behandelten Flächen zu anderen Personen zu meiden. In der Zeit zwischen 7.30 Uhr und 8.00 Uhr, vor Applikation des Gels wurde jedem Probanden eine Blutprobe zur Bestimmung des Hormonausgangswerts entnommen. Danach wurden im Abstand von 1, 3 und 6 Stunden (9.00 Uhr, 11.00 Uhr und 14.00 Uhr) Blutproben zur Ermittlung der Testosteron-Serumwerte entnommen.

Die Ergebnisse der durchgeführten Hormonanalysen sind in Fig. 1 dargestellt. Der mittlere Testosteronausgangswert betrug 14,0 nmol/l. Die mittleren Testosteronserumwerte nach 1, 3 und 6 Stunden betrugen 29,5, 22,7, bzw. 20,0 nmol/l. Die senkrechten Strecken in Fig. 1 zeigen die Streuung der jeweiligen Serumwerte infolge eines unterschiedlich verlaufenden Anstiegs und anschließenden Abfall der Testosteronwerte bei den Probanden.

Durch die transskrotale Applikation ist es möglich, die zur Normalisierung des Grenzwertes bei Hypogonadismus führende Gelmenge bzw. Testosterondosis deutlich zu reduzieren und die vorstehend beschriebenen Nachteile zu vermeiden.

### Beispiel 2

Zum Vergleich wurden in einer analog durchgeführten Untersuchung bei sechs weiteren Probanden 5 g Gel im Brust-, Bauch- oder Oberarmbereich aufgetragen (anstatt 1 g Gel auf das Skrotum). Für die ermittelten mittleren Testosteron-Serumwerte konnte gegenüber den in Fig. 1 dargestellten Werten keine signifikante Abweichung festgestellt werden.

### Beispiel 3

Das in Beispiel 1 hergestellte Gel G-II mit einem Testosterongehalt von 2,5 Gew.-% wurde in einer Pilotstudie hinsichtlich seiner pharmakokinetischen und pharmakodynamischen Eigenschaften untersucht. Diese Studie hatte das Ziel, den über 24 Stunden gemessenen Einfluss des Testosterongels auf den Serumspiegel über einen Zeitraum von 10 Applikationstagen zu überprüfen. Darüber hinaus wurde die Möglichkeit des Gel-Aabwaschens nach 10 Minuten Einwirkdauer und die nach Hautkontakt ins Blut gehende Testosteronmenge untersucht.

14 männliche Probanden applizierten täglich 5 g des Testosterongels auf den Oberkörper. Von diesen Probanden wuschen sieben das Gel jeweils 10 Minuten nach der Applikation ab.

Zielparameter für die Messung des resorbierten Testosterons waren die Serumtagesspiegel im Zeitverlauf von 24 Stunden und 10 Tagen. Als Messkritierien dienten die Fläche unter der Konzentrations-Zeit-Kurve (AUC₀₋₂₄) und der maximale Serumspiegel (Cₘₐₓ) sowie die Zeitdauer des durch die Gelapplikation erhöhten Serumspiegels (t).

Mit dem eingesetzten Testosterongel konnte eine adäquate Testosteronsubstitution gewährleistet werden. Ein supraphysiologischer Anstieg von Östradiol und DHT wurde nicht beobachtet. Eine Akkumulation des Testosteronserumspiegels vom ersten bis zum zehnten Tag wurde beobachtet (δCₘₐₓ Tag 1: 10,0 nmol/l ± 8,8 MW ± Sdev; Tag 5: 17,9 nmol/l ± 10,0; Tag 10: 20,9 nmol/l ± 13,6). Das Waschen der Haut nach 10 Minuten führte nicht zu einer schlechteren Resorption des Testosterongels.

Damit beeinflusst ein Waschen der Haut 10 Minuten nach Applikation des Testosterongels nicht das pharmakokinetische Profil und verringert dadurch noch wirksamer die Möglichkeit einer Übertragung von Testosteron auf Dritte.

## Patentansprüche

1. Verwendung einer Gelzusammensetzung auf alkoholischer Basis, umfassend 0,1 bis 4 Gew.-% mindestens eines androgenen Steroids, 0,1 bis 20 Gew.-% mindestens eines C₃-C₄-Diols als Resorptionsverstärker, 30 bis 90 Gew.-% mindestens eines C₂-C₇-Alkohols und Mischungen davon, 0,01 bis 1 Gew.-% mindestens eines pH-Stabilisatoren, 0,1 bis 6 Gew.-% mindestens eines Polymeren auf Poly(meth)acrylsäurebasis, sowie optional Hilfsstoffe, ausgewählt unter Konservierungsstoffen, Antioxidantien, Stabilisatoren, Lösungsvermittlern, Vitaminen, Färbemitteln und Geruchsverbesserungen, zur Herstellung eines Gels zur Behandlung und/oder Prophylaxe von Hypogonadismus durch transkrotale Applikation.

2. Verwendung nach Anspruch 1, **dadurch gekennzeichnet, dass** das androgene Steroid ausgewählt ist aus der Gruppe bestehend aus Testosteron, Testosteronestern, Methyltestosteron, Dihydrotestosteron und Gemischen davon.

3. Verwendung nach Anspruch 1 oder 2, **dadurch gekennzeichnet, dass** die Zusammensetzung in einer Menge von 0,2 bis 20 g verwendet wird.

4. Verwendung nach Anspruch 1, 2 oder 3, **dadurch gekennzeichnet, dass** die Zusammensetzung in einer Menge von 1,0 bis 7 g verwendet wird.

5. Verwendung nach einem der vorstehenden Ansprüche, **dadurch gekennzeichnet, dass** das C₃-C₄-Diol Propylenglykol ist.

6. Verwendung nach einem der vorstehenden Ansprüche, **dadurch gekennzeichnet, dass** der Alkohol ausgewählt wird aus der Gruppe, bestehend aus C₂-C₄-Alkoholen sowie Mischungen davon.

7. Verwendung nach einem der vorstehenden Ansprüche,**dadurch gekennzeichnet, dass** der Alkohol Ethanol oder Propanol ist.

8. Verwendung nach einem der vorstehenden Ansprüche, **dadurch gekennzeichnet, dass** der mindestens eine pH-Stabilisator Tromethamol ist

## Claims

1. Use of a gel composition on alcoholic basis, comprising 0.1 to 4 wt% of at least one androgenic steroid, 0.1 to 20 wt% of at least one C₃-C₄ diol as a resorption enhancer, 30 to 90 wt.% of at least one C₂-C₇ alcohol and mixtures thereof, 0.01 to 1 wt% of at least one pH stabilizer, 0.1 to 6 wt% of at least one polymer on the basis of poly(meth)acrylic acid, as well as optional excipients, selected among preservatives, antioxidants, stabilizers, solution enhancers, vitamins, colouring agents and odour-improving agents, for the preparation of a gel for the treatment and/or prophylaxis of hypogonadism by transscrotal application.

2. Use in accordance with claim 1, **characterised in that** the androgenic steroid is selected from the group consisting of testosterone, testosterone esters, methyl testosterone, dihydrotestosterone, and mixtures thereof.

3. Use in accordance with claim 1 or 2, **characterised in that** the composition is used in an amount from 0.2 to 20 g.

4. Use in accordance with claim 1, 2 or 3, **characterised in that** the composition is used in an amount from 1.0 to 7 g.

5. Use in accordance with any one of the preceding claims, **characterised in that** the C₃-C₄ diol is propylene glycol.

6. Use in accordance with any one of the preceding claims, **characterised in that** the alcohol is selected from the group consisting of C₂-C₄ alcohols as well as mixtures thereof.

7. Use in accordance with any one of the preceding claims, **characterised in that** the alcohol is ethanol or propanol.

8. Use in accordance with any one of the preceding claims, **characterised in that** the at least one pH-stabilizer is tromethamol.

## Revendications

1. Utilisation d'une composition en gel à base alcoolique, comprenant 0,1 à 4 % en poids d'au moins un stéroïde androgène, 0,1 à 20 % en poids d'au moins un diol en C₃-C₄ à titre d'agent renforçant la résorption, 30 à 90 % en poids d'au moins un alcool en C₂-C₇ et des mélanges de ceux-ci, 0, 01 à 1 % en poids d' au moins un agent stabilisant le pH, 0,1 à 6 % en poids d'au moins un polymère à base d'acide poly(méth) acrylique, ainsi que des auxiliaires éventuels, choisis parmi les conservateurs, les antioxydants, les stabilisants, les agents favorisant la dissolution, les vitamines, les colorants et les améliorants olfactifs, pour la fabrication d'un gel pour le traitement et/ou la prophylaxie de l'hypogonadisme par application transcrotale.

2. Utilisation selon la revendication 1, **caractérisée en ce que** le stéroïde androgène est choisi dans le groupe constitué de la testostérone, des esters de la testostérone, de la Méthyl testostérone, de la dihydrotestostérone et de leurs mélanges.

3. Utilisation selon la revendication 1 ou 2, **caractérisée en ce que** la composition est utilisée en une quantité de 0,2 à 20 g.

4. Utilisation selon la revendication 1, 2 ou 3, **caractérisée en ce que** la composition est utilisée en une quantité de 1,0 à 7 g.

5. Utilisation selon l'une des revendications précédentes, **caractérisée en ce que** le diol en C₃-C₄ est le propylène glycol.

6. Utilisation selon l'une des revendications précédentes, **caractérisée en ce que** l'alcool est choisi dans le groupe constitué des alcools en C₂-C₄ et de leurs mélanges.

7. Utilisation selon l'une des revendications précédentes, **caractérisée en ce que** l'alcool est l'éthanol ou le propanol.

8. Utilisation selon l'une des revendications précédentes, **caractérisée en ce que** le au moins un agent stabilisant le pH est le trométhamol.
